(19) Europäisches Patentamt
European Patent Office
Office européen des brevets

(11) Veröffentlichungsnummer: **0 176 925**
**A2**

(12) # EUROPÄISCHE PATENTANMELDUNG

(21) Anmeldenummer: **85112069.1**

(22) Anmeldetag: **24.09.85**

(51) Int.Cl.[4]: **C 07 D 239/52**
C 07 D 239/34, C 07 D 239/56
C 07 D 239/70
//A01N47/18

(30) Priorität: **05.10.84 DE 3436478**

(43) Veröffentlichungstag der Anmeldung:
**09.04.86 Patentblatt 86/15**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Maurer, Fritz, Dr.**
**Roeberstrasse 8**
**D-5600 Wuppertal 1(DE)**

(54) **Verfahren zur Herstellung von N,N-Dimethyl-O-pyrimidinyl-carbaminsäureestern.**

(57) Es wird ein neues Verfahren zur Herstellung von N,N-Dimethyl-O-pyrimidinylcarbaminsäureestern der Formel (I)

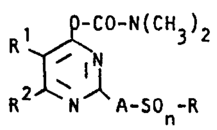

(I)

bereitgestellt,
in welcher
R, $R^1$, $R^2$, A und n die im Anmeldungstext angegebene Bedeutung haben.

Das neue Verfahren ist dadurch gekennzeichnet, daß man Hydroxypyrimidine der Formel (II)

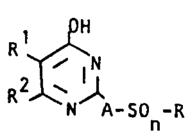

(II)

in welcher
R, $R^1$, $R^2$, A und n die im Anmeldungstext angegebene Bedeutung haben,
mit N,N-Dimethyl-carbaminsäurehalogeniden der Formel (III)

$Hal-CO-N(CH3)2$ (III)

in welcher
Hal für Chlor oder Brom steht,
in Gegenwart katalytischer Mengen eines bicyclischen organischen Amins in Gegenwart von Säureakzeptoren und in Gegenwart eines Verdünnungsmittels bei Temperaturen zwischen 0°C und 150°C und insbesondere zwischen 20°C und 100°C umsetzt.

Als bicyclisches organisches Amin wird vorzugsweise 1,4-Diazabicyclo (2,2,2)-octan eingesetzt.

EP 0 176 925 A2

Croydon Printing Company Ltd

BAYER AKTIENGESELLSCHAFT 5090 Leverkusen, Bayerwerk
Konzernverwaltung RP
Patentabteilung Er/ABc
IVa

Verfahren zur Herstellung von N,N-Dimethyl-O-pyrimidinylcarbaminsäureestern

Die Erfindung betrifft ein neues Verfahren zur Herstellung von N,N-Dimethyl-O-pyrimidinyl-carbaminsäureestern aus Hydroxypyrimidinen in Gegenwart eines bicyclischen Amins als Katalysator.

Es ist bereits bekannt, daß man N,N-Dimethyl-O-pyrimidinylcarbaminsäureester erhält, wenn man Hydroxypyrimidine in Gegenwart von anorganischen Basen wie z.B. Natrium- oder Kaliumcarbonat mit N,N-Dimethyl-carbaminsäurehalogeniden umsetzt (vgl. DE-OS 2 928 185).

Die Nachteile dieses Verfahrens bestehen darin, daß sehr lange Verweilzeiten erforderlich sind, große Mengen an isomeren Nebenprodukten entstehen und die Ausbeuten dadurch häufig sehr unbefriedigend sind.

In der DE-OS 3 211 035 (siehe dortige Verfahrensvariante a)) wird erwähnt, daß die Herstellung von N,N-Dimethyl-O-pyrimidinylcarbaminsäureestern aus Hydroxypyrimidinen

und N,N-Dimethylcarbamoylhalogeniden in Gegenwart von Diazabicyclooctan, Diazabicyclononan und Diazabicycloundecan als Säureakzeptoren möglich ist. Siehe dazu Seite 32, letzter Absatz der vorgenannten Offenlegungsschrift.

Der Einsatz katalytischer Mengen eines bicyclischen organischen Amins (corzugsweise von 1,4-Diazabicyclo (2,2,2)-octan) neben molaren Mengen eines Säueakzeptors wird in der DE-OS 3 211 035 weder erwähnt noch nahegelegt.

Es wurde nun gefunden, daß man N,N-Dimethyl-O-pyrimidinyl-carbaminsäureester der Formel (I)

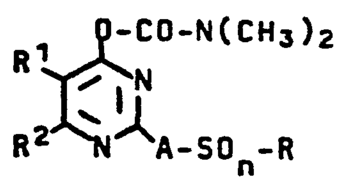

(I)

in welcher

R für geradkettiges oder verzweigtes Alkyl steht,

$R^1$ für Wasserstoff oder gegebenenfalls substituierte Reste aus der Reihe Alkyl, Alkoxy, Alkylthio, Alkylsulfinyl oder Alkylsulfonyl steht,

$R^2$ für Wasserstoff oder gegebenenfalls substituiertes Alkyl steht oder

$R^1$ und $R^2$ gemeinsam einen ankondensierten gegebenenfalls substituierten gesättigten oder ungesättigten Ring bilden können,

A für geradkettiges oder verzweigtes Alkylen steht und

n für 0, 1 oder 2 steht,

erhält,

wenn man Hydroxypyrimidine der Formel (II)

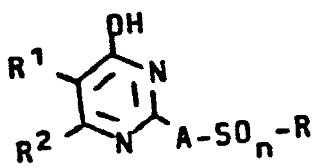

(II)

in welcher

R, $R^1$, $R^2$, A und n die oben genannten Bedeutungen haben,

mit N,N-Dimethyl-carbaminsäurehalogeniden der Formel (III)

$$Hal-CO-N(CH_3)_2 \qquad (III)$$

in welcher

Hal für Chlor oder Brom steht,

in Gegenwart katalytischer Mengen eines bicyclischen organischen Amins, in Gegenwart von Säureakzeptoren und in Gegenwart eines Verdünnungsmittels bei Temperaturen zwischen 0°C und 150°C und insbesondere zwischen 20°C und 100°C umsetzt.

Als bicyclische organische Amine kommen bevorzugt 1,4-Diazabicyclo-(2,2,2)-octan (DABCO) und Chinuclidin in Frage, insbesondere bevorzugt ist DABCO.

Überraschenderweise ist es möglich, mit Hilfe des erfindungsgemäßen Verfahrens die Umsetzung mit sehr kurzen Verweilzeiten in hoher Ausbeute und Reinheit durchzuführen. Dies ist umso überraschender, als nach dem Stand der Technik die Bildung von isomeren Nebenprodukten in beträchtlichem Ausmaß hätte erwartet werden müssen.

Bevorzugt werden mit dem erfindungsgemäßen Verfahren die folgenden Verbindungen der Formel (I) hergestellt, in welcher

R für geradkettiges oder verzweigtes Alkyl mit 1 bis 6 Kohlenstoffatomen, insbesondere 1 bis 4 Kohlenstoffatomen, steht,

$R^1$ für Wasserstoff oder für Alkyl, Alkoxy oder Alkylthio, Alkylsulfinyl oder Alkylsulfonyl, jeweils mit 1 bis 6 Kohlenstoffatomen, insbesondere 1 bis 4 Kohlenstoffatomen, die gegebenenfalls Halogen oder $C_1$-$C_2$-Alkoxy substituiert sein können, steht,

$R^2$ für Wasserstoff oder Alkyl mit 1 bis 6 Kohlenstoffatomen, insbesondere 1 bis 4 Kohlenstoffatomen, das gegebenenfalls durch Halogen, insbesondere Chlor oder Fluor oder $C_1$-$C_2$-Alkoxy substituiert sein kann, steht oder

$R^1$ und $R^2$ gemeinsam für Alkylen mit 2 bis 4 Kohlenstoffatomen, das gegebenenfalls durch Halogen, insbesondere Chlor oder Fluor oder $C_1$-$C_2$-Alkyl substituiert sein kann, stehen,

A für geradkettiges oder verzweigtes Alkylen mit 1 bis 6 Kohlenstoffatomen, insbesondere 1 bis 4 Kohlenstoffatomen, steht und

n für 0, 1 oder 2 steht.

Verwendet man beispielsweise für das erfindungsgemäße Verfahren 4-Hydroxy-5-methoxy-2-methylthiomethyl-pyrimidin und N,N-Dimethylcarbaminsäurechlorid als Ausgangsstoffe, in Gegenwart von katalytischen Mengen 1,4-Diazabicyclo-(2,2,2)-octan (DABCO), so kann die Reaktion durch das folgende Formelschema skizziert werden:

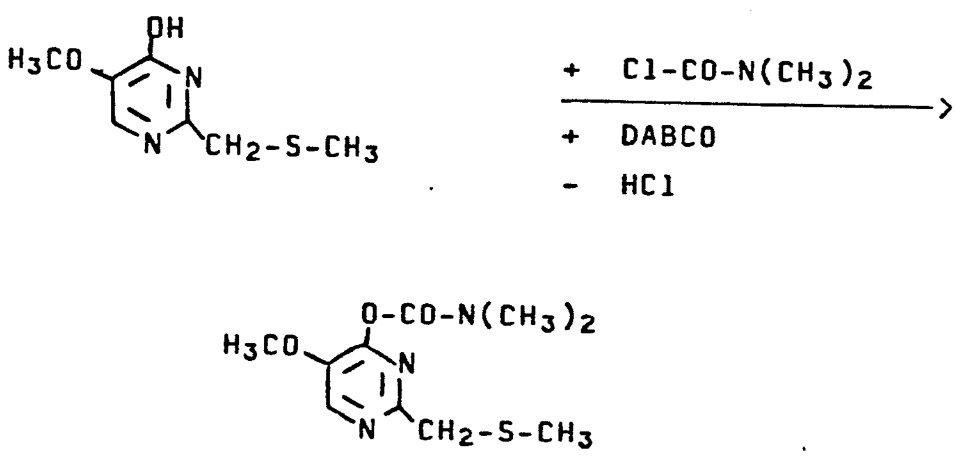

Die zur Durchführung des erfindungsgemäßen Verfahrens als Ausgangsstoffe zu verwendenden Hydroxypyrimidine sind durch Formel (II) allgemein definiert. Vorzugsweise stehen darin R, $R^1$, $R^2$, A und n für diejenigen Reste und Indices, welche bei der Definition von R, $R^1$, $R^2$, A und n in Formel (I) als bevorzugt genannt wurden.

Die Verbindungen der Formel (II) sind bekannt und/oder können nach an sich bekannten Methoden hergestellt werden (vgl. DE-OS 2 838 359, DE-OS 2 928 185 und DE-OS 3 211 035).

Die Verbindungen der Formel (III) sind bekannte Verbindungen der organischen Chemie. Als Beispiel sei N,N-Dimethylcarbaminsäurechlorid genannt.

Das erfindungsgemäße Verfahren zur Herstellung von N,N-Dimethyl-O-pyrimidinyl-carbaminsäureestern wird im allgemeinen unter Verwendung von Verdünnungsmitteln durchgeführt. Als solche kommen praktisch alle inerten organischen Lösungsmittel in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether wie Diethyl- und Dibutylether, Tetrahydrofuran und Dioxan, Ketone wie Aceton, Methylethyl-, Methylisopropyl- und Methylisobutylketon sowie Nitrile wie Acetonitril und Propionitril.

Das erfindungsgemäße Verfahren wird unter Verwendung von Säureakzeptoren durchgeführt. Als Säureakzeptoren

können alle üblichen Säurebindemittel Verwendung finden. Besonders bewährt haben sich Alkalicarbonate, wie Natrium- und Kaliumcarbonat, ferner aliphatische, aromatische oder heterocyclische Amine, beispielsweise Triethylamin, Trimethylamin, Dimethylanilin, Dimethylbenzylamin und Pyrimidin.

Das erfindungsgemäße Verfahren wird in Gegenwart eines bicyclischen organischen Amins und insbesondere in Gegenwart von 1,4-Diazabicyclo-(2,2,2)-octan (DABCO) als Katalysator durchgeführt.

In einer bevorzugten Ausführungsform werden als Säureakzeptoren Alkalicarbonate insbesondere $Na_2CO_3$ und/oder $K_2CO_3$ und katalytische Mengen von DABCO eingesetzt.

Das erfindungsgemäße Verfahren wird im allgemeinen bei Temperaturen zwischen 0°C und 150°C durchgeführt. Bevorzugt ist der Temperaturbereich zwischen 20°C und 100°C. Die Umsetzungen werden im allgemeinen bei Normaldruck durchgeführt.

Zur Durchführung des erfindungsgemäßen Verfahrens werden auf 1 Mol Hydroxy-pyrimidin der Formel (II) 1,0 bis 1,3 Mol, vorzugsweise zwischen 1,0 und 1,2 Mol N,N-Dimethylcarbaminsäurechlorid und 0,01 bis 0,08 Mol, vorzugsweise zwischen 0,02 bis 0,06 Mol bicyclisches organisches Amin und insbesondere von DABCO eingesetzt. Die Umsetzung wird in einem Verdünnungsmittel in Gegenwart eines Säureakzeptors durchgeführt. Nach Ablauf der Reaktion wird filtriert und das Lösungsmittel im Vakuum abdestilliert.

Die Verbindungen der Formel (I) fallen in der Regel in fester Form an und können durch Umkristallisation gereinigt werden. Zur Charakterisierung dient der Schmelzpunkt.

Die nach dem erfindungsgemäßen Verfahren hergestellten N,N-Dimethyl-O-pyrimidinyl-carbaminsäureester sind hochwirksame Insektizide (vgl. DE-OS 2 838 359, DE-OS 2 928 185 und DE-OS 3 211 035).

Herstellungsbeispiele

Beispiel 1

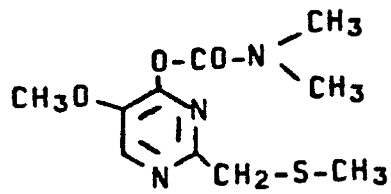

Zu einer Mischung aus 19,5 g (0,1 Mol) 95,3 proz. 2-Methylthiomethyl-4-hydroxy-5-methoxypyrimidin, 100 ml Chloroform, 13,2 g (0,125 Mol) wasserfreiem Natriumcarbonat und 0,56 g (0,005 Mol) Diazabicyclo(2,2,2)-octan gibt man 11,65 g (0,103 Mol) 95 proz. N,N-Dimethylcarbamidsäurechlorid. Die Temperatur der Reaktionsmischung steigt bis auf ca. 34°C an und fällt dann langsam wieder ab. Nach 1 1/2 Stunden ist kein Vorprodukt mehr vorhanden. Das anorganische Salz wird abgesaugt, mit Chloroform wird nachgewaschen und anschließend wird das Lösungsmittel im Vakuum entfernt.

Man erhält 27,3 g 93 prozentigen N,N-Dimethyl-O-(2-methyl-thiomethyl-5-methoxypyrimidin-4-yl)-carbaminsäureester (99 % der Theorie) in Form beiger Kristalle mit dem Schmelzpunkt 96°C.

Auf die gleiche Weise erhält man z.B. folgende Verbindung:

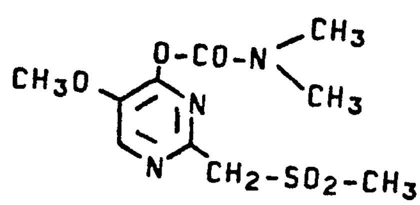

Ausbeute: 99 % der Theorie
Fp: 118°C

Vergleichsbeispiel aus DE-OS 2 928 185

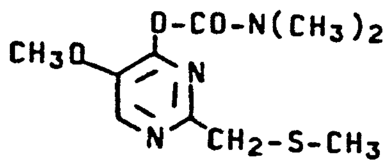

Ein Gemisch aus 18,6 g (0,1 Mol) 2-Methylthiomethyl-4-hydroxy-5-methoxypyrimidin, 16,6 g (0,12 Mol) Kaliumcarbonat, 200 ml Acetonitril und 10,8 g (0,1 Mol) Dimethylcarbaminsäurechlorid wird 7 Stunden unter Rückfluß gekocht, auf Raumtemperatur abgekühlt und dann filtriert. Das Filtrat wird im Vakuum eingedampft.

Man erhält 19,5 g (76 % d.Th.) N,N-Dimethyl-O-(2-methylthiomethyl-5-methoxypyrimidin-4-yl)-carbaminsäureester in Form beiger Kristalle mit dem Schmelzpunkt 97°C.

Patentansprüche

1. Verfahren zur Herstellung von N,N-Dimethyl-O-pyrimidinyl-carbaminsäureester der Formel (I)

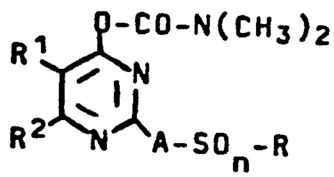

(I)

in welcher

R für geradkettiges oder verzweigtes Alkyl steht,

$R^1$ für Wasserstoff oder gegebenenfalls substituierte Reste aus der Reihe Alkyl, Alkoxy, Alkylthio, Alkylsulfinyl oder Alkylsulfonyl steht,

$R^2$ für Wasserstoff oder gegebenenfalls substituiertes Alkyl steht oder

$R^1$ und $R^2$ gemeinsam einen ankondensierten gegegebenenfalls substituierten gesättigten oder ungesättigten Ring bilden können,

A für geradkettiges oder verzweigtes Alkylen steht und

n für 0, 1 oder 2 steht,

durch Umsetzung von Hydroxypyrimidinen der Formel (II)

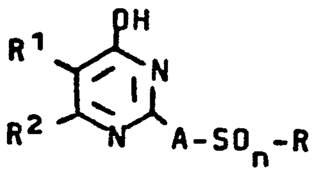

(II)

in welcher

R, $R^1$, $R^2$, A und n die oben angegebene Bedeutungen haben,

mit N,N-Dimethyl-carbaminsäurehalogeniden der Formel (III)

$$Hal-CO-N(CH_3)_2 \qquad (III)$$

in welcher

Hal für Chlor oder Brom steht,

in Gegenwart von Säureakzeptoren, dadurch gekennzeichnet, daß man die Umsetzung in Gegenwart katalytischer Mengen eines bicyclischen organischen Amins und in Gegenwart eines Verdünnungsmittels durchführt.

2. Verfahren gemäß Anspruch 1), dadurch gekennzeichnet, daß man die Umsetzung bei Temperaturen zwischen 0°C und 150°C durchführt.

3. Verfahren gemäß Anspruch 1)-2), dadurch gekennzeichnet, daß man die Umsetzung bei Temperaturen zwischen 20°C und 100°C durchführt.

4. Verfahren gemäß Anspruch 1)-3), dadurch gekennzeichnet, daß man als bicyclisches organisches Amin die Verbindung 1,4-Diazabicyclo-(2,2,2)-octan einsetzt.

5. Verfahren gemäß Anspruch 1) bis 3) zur Herstellung von Verbindungen der Formel (I), in welcher

R für geradkettiges oder verzweigtes Alkyl mit 1 bis 6 Kohlenstoffatomen, insbesondere 1 bis 4 Kohlenstoffatomen, steht,

$R^1$ für Wasserstoff oder für Alkyl, Alkoxy oder Alkylthio, Alkylsulfinyl oder Alkylsulfonyl, jeweils mit 1 bis 6 Kohlenstoffatomen, insbesondere 1 bis 4 Kohlenstoffatomen, die gegebenenfalls Halogen oder $C_1$-$C_2$-Alkoxy substituiert sein können, steht,

$R^2$ für Wasserstoff oder Alkyl mit 1 bis 6 Kohlenstoffatomen, insbesondere 1 bis 4 Kohlenstoffatomen, das gegebenenfalls durch Halogen, insbesondere Chlor oder Fluor oder $C_1$-$C_2$-Alkoxy substituiert sein kann, steht oder

$R^1$ und $R^2$ gemeinsam für Alkylen mit 2 bis 4 Kohlenstoffatomen, das gegebenenfalls durch Halogen, insbesondere Chlor oder Fluor oder $C_1$-$C_2$-Alkyl substituiert sein kann, stehen,

A für geradkettiges oder verzweigtes Alkylen mit 1 bis 6 Kohlenstoffatomen, insbesondere 1 bis 4 Kohlenstoffatomen, steht und

n für 0, 1 oder 2 steht.

6. Verfahren gemäß Anspruch 1) bis 5) zur Herstellung von N,N-Dimethyl-O-(2-methylthiomethyl-5-methoxy-pyrimidin-4-yl)-carbaminsäureester.

7. Verfahren gemäß Anspruch 1) bis 6), dadurch gekennzeichnet, daß man pro Mol Hydroxypyrimidin der Formel II 1,0 bis 1,3 Mol N,N-Dimethyl-carbaminsäurechlorid und 0,01 bis 0,08 Mol 1,4-Diazabicyclo-(2,2,2)-octan einsetzt.

8. Verfahren gemäß Anspruch 1) bis 7), dadurch gekennzeichnet, daß man pro Mol Hydroxypyrimidin der Formel II 1,0 bis 1,2 Mol N,N-Dimethylcarbaminsäurechlorid und 0,02 bis 0,06 Mol 1,4-Diazabicyclo-(2,2,2)-octan einsetzt.

9. Verfahren nach Anspruch 1), dadurch gekennzeichnet, daß es sich bei dem Verdünnungsmittel um ein inertes organisches Lösungsmittel handelt.